# EUROPÄISCHE PATENTSCHRIFT

(11) **EP 2 214 626 B1**
(45) Veröffentlichungstag und Bekanntmachung des Hinweises auf die Patenterteilung: **12.10.2011**
(21) Anmeldenummer: 08845140.6
(22) Anmeldetag: 27.10.2008
(51) Int. Cl.: A61K 8/26, A61K 8/20, A61K 8/92, A61Q 5/12, A61Q 9/02, A61Q 15/00

(54) **VERMINDERUNG DER RASURFREQUENZ**
REDUCING SHAVING FREQUENCY
DIMINUTION DE LA FRÉQUENCE DE RASAGE

(30) Priorität: 02.11.2007 DE 102007052865
(43) Veröffentlichungstag der Anmeldung: 11.08.2010
(73) Patentinhaber: Beiersdorf AG, 20253 Hamburg (DE)
(72) Erfinder: WILKE, Katrin, 21644 Sauensiek (DE); WINDISCH, Björna, 22607 Hamburg (DE); MAURER, Peter, 24536 Neumünster (DE); MÜLLER, Claudia, 25499 Tangstedt (DE); LIPINSKI, Norman, 60322 Frankfurt (DE); URBAN, Michael, 22523 Hamburg (DE); MÜHLEN, Nicole, 22946 Trittau (DE); HEINS, Sabine, 25462 Rellingen (DE); MANNES, Annika, 22459 Hamburg (DE)
(74) Vertreter: Hartmann, Jost
(86) Internationale Anmeldenummer: PCT/EP2008/009066
(87) Internationale Veröffentlichungsnummer: WO 2009/056267

(56) Entgegenhaltungen:
- DE-A1-102004 049 282
- DE-A1-102005 028 384
- US-A- 2 145 583
- US-A- 4 551 330
- US-A- 5 217 652
- US-A1- 2003 165 447
- US-A1- 2004 120 909
- US-A1- 2004 253 187
- US-A1- 2006 140 891
- US-B1- 6 495 149

## Beschreibung

Die Erfindung beschreibt die Verwendung saurer Aluminiumverbindungen, die in kosmetischen Zubereitungen zur Haarerweichung, zur Schneidkraftreduktion und zur Verringerung der Frequenz der Rasur führen.

Die Körper- und insbesondere Hautpflege umfasst neben der Hautreinigung, der Hautpflege auch zunehmend die Körperrasur.

Neben den Barthaaren des Mannes werden sowohl von Männern als auch Frauen die Bein- und vor allem die Achselbehaarung regelmäßig entfernt.

Zu diesem Zwecke sind verschiedenste Mittel, wie Epilationsmittel, und Zubereitungen bekannt.

Kern aller bekannten Mittel ist die Hautglätte zu steigern und möglichst lange glatte Haut zu bewahren. Dabei ist es wünschenswert die Rasurfrequenz, d.h. die Häufigkeit der Haarentfemung, zu minimieren.

Dies vor allem deswegen, da bei der Rasur oder Haarentfernung die Haut häufig gereizt wird. Eine tägliche Haarentfemung wird damit mitunter unmöglich oder ist mit starken Hautirritationen verbunden.

Häufiges Rasieren insbesondere im Achselbereich führt erfahrungsgemäß zu Hautreizungen und Hautirritationen. Der Stand der Technik versuchte diesem Misstand durch haarwuchsreduzierende Mittel zu beheben.

Beispielsweise ist Papaya als haarwuchsreduzierendes Mittel in den Schutzrechten WO 2006125582 beschrieben.

WO 200730139 beschreibt kosmetische Zubereitungen umfassend Behenoxy dimethicone. Diese Zubereitungen sollen eine verbesserte Hautverträgtichkeit und die Erkennbarkeit von Haaren und Bartstoppeln verringern helfen.

EP 1588690 beschreibt Mischungen und Verfahren zur Verzögerung oder Verhinderung von unerwünschtem Haarwuchs, wobei die Mischung Äpfelsäure, einen Elektrolyten und ein in der Kosmetik akzeptables, auf Wasser basierendes Mittel, das ein filmbildendes Mittel einschliesst, aufweist.

In der US 7238343 B2 werden Deodorantien und Antitranspirantien beschrieben, die natürliche Öle, insbesondere Sonnenblumenöl, und Glycerin im Verhältnis 4:1 bis 1:4 enthalten. Die Verwendung derartiger Zubereitungen soll die Regeneration der Haut nach der Haarentfemung verbessern.

In der WO 2006125582 A1 werden Antitranspirant-Emulsionen mit Haarwuchsinhibitoren beschrieben.

Rasiermittel, Schäume und Gele basieren auf basischen Zubereitungen, da bekanntermaßen die seifenbasierten und damit alkalischen Zubereitungen zu einem Quellen der Haaren führen, und damit eine gründlichere Rasur stattfinden kann.

Den o.g. Rasurzubereitungen werden zuweilen Rasurhilfsmittel zugesetzt. Dabei handelt es sich um partikuläre Verbindungen oder wasserlösliche Polymere, wie PEG-14M, PEG-7M, PEG-90M (=PEG 90000), PEG 45M (Polymere des Ethylenoxids mit durchschnittlichen Polymerisationsgraden wie beziffert multipliziert mit Faktor 1000), Acrylate, PTFE (Polytetrafluorethylene) oder Cellulose-Derivate, die geeignet sind das Gleitvermögen zu verbessern.

Bekannte kosmetische Rasurzubereitungen haben häufig eine gleitverbessernde Substanz aus der Gruppe der ethoxylierten Polywachse, wie z.B. PEG-6000 als Inhaltststoff. Der Einsatz von PEG Verbindungen in kosmetischen Präparaten wird aber sehr kontrovers diskutiert. Als Grund für die Bedenken wird unter anderem die Möglichkeit einer Penetrationsförderung für potenziell irritative oder allergene Bestandteile der Kosmetika angeführt.

Bei den PEG-Verbindungen handelt es sich um eine große Gruppe von sehr unterschiedlichen Substanzen, die hinsichtlich ihrer penetrationsfördernden Eigenschaften nicht einheitlich sind.

Aufgabe der vorliegenden Erfindung ist es daher kosmetische Rasurzubereitungen bereit zu stellen, die die Haut unter den Achseln auf die Rasur vorbereiten und das Gleit - und Schneidvermögen bei der Rasur verbessern.

Wünschenswert ist es dabei auf penetrationsfördernde Bestandteile zu verzichten oder deren Anteil zu verringern.

Bei den Rasurzubereitungen des Standes der Technik ist zudem häufig eine gute Hautverträglichkeit nicht gegeben.

US 6495149 B1 als auch US 2003165447 A1 beschreiben Antitranspirant- und Deodorantzubereitungen in denen Pantothensäure bzw. deren Derivate einen konditionierenden oder erweichenden Effekt auf das Achselhaar ausüben und somit deren Anwendung zu einer gründlicheren Rasur führt.

Weiteres Manko ist, dass verschiedenste Mittel erhältlich sind. Also neben den Rasurhilfsmitteln auch zu verwendenden Deo- bzw. Antitranspirantprodukte, die parallel oder hintereinander anzuwenden sind.

Neben der Haarwuchsreduktion ist vor allem die Hautverträglichkeit ein entscheidendes Merkmal effizienter und vom Kunden akzeptierter Deo/AT-mittel.

Die Erfindung geht nun einen anderen Weg, bei der nicht die Haarwuchsreduktion sondern die Haarrasurfrequenz und die Hautverträglichkeit im Vordergrund stehen.

Überraschenderweise hat sich gezeigt, dass saure Aluminiumverbindungen in kosmetischen Zubereitungen zur Verringerung der Rasurfrequenz beitragen.

Es werden erfindungsgemäß saure Aluminiumverbindungen in topisch applizierbaren Zubereitungen zur Verringerung der Rasurfrequenz verwendet.

Weiterhin werden saure Aluminiumverbindungen in topisch applizierbaren Zubereitungen zur Erweichung des menschlichen Haares, insbesondere der Achselbehaarung, erfindungsgemäß verwendet.

Die Verwendung von sauren Aluminiumverbindungen in Zubereitungen führt zur Erniedrigung der Rasurschneidkraft nach topischer Applikation der Zubereitung.

Eine besondere Bedeutung auf die kosmetische Rasurzubereitung hat auch die Gleitkraft, mit der die Klinge auf der Hautoberfläche fortbewegt wird. Diese soll möglichst niedrig sein.

Ausserdern sollte die Zubereitung gut auf die Haut aufziehen und weniger gut auf den Rasierer, damit dieser nicht verklebt oder schneller abstumpft. Die Rasurzubereitung sollte auch das Haar stabilisieren, d.h. in einer Form aufrichten, so dass es tiefer abgeschnitten werden kann. Gerade nach diesem frischen Anschnitt sollte die irritierte, mechanisch gereizte Haut, gepflegt und beruhigt werden.

Genau diese Fülle an Aufgaben löst die erfindungsgemäße Rasurzubereitung.

Vergleichstests belegen, dass saure Aluminiumverbindungen, wie beispielsweise Aluminiumchlorohydrate, zu einer Schneidkrafterniedrigung des Haares bei der Rasur führen.

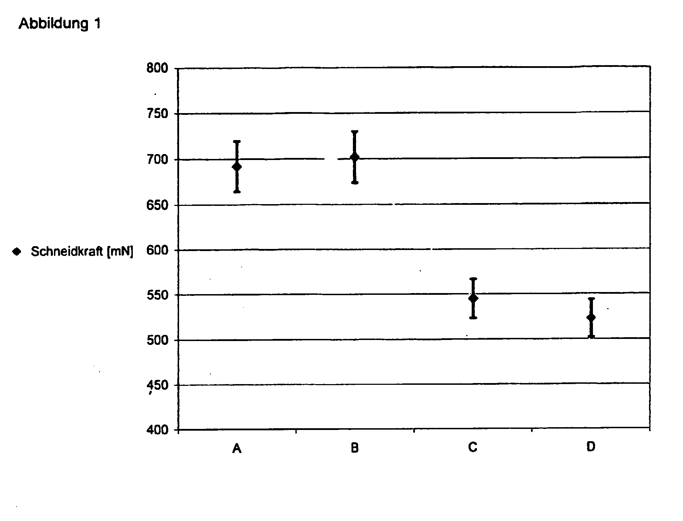

Abbildung 1 zeigt die Schneidkraft bei der Rasur menschlichen Haars nach 24 stündiger Einwirkzeit der Produkte A bis D.

Das Gleiten der Rasierklinge kann physikalisch in Trocken- und Flüssigkeitsreibung unterteilt werden. Die coloumbsche Trockenreibung wird dabei von dem Reibungskoeffizient des Untergrundes beeinflusst, die viskose Reibung zwischen bzw. das Gleiten von Flüssigkeitsfilmen wird dabei maßgeblich durch die dynamische Viskosität bestimmt.

Auch die Schneidkraft, also die Kraft, die erforderlich ist, um ein Haar abzuschneiden ist beim Rasieren von besonderer Bedeutung.

Beim Schneiden handelt es sich bezogen auf die Werkstofftechnik um ein Trennverfahren. Das Schneiden eines Bartstoppels wird dabei am besten durch das Prinzip des Keilschneidens beschrieben. Die Schneidkraft FB_{SB} kann dabei mit Hilfe der folgenden Formel beschrieben werden:

| | |
|---|---|
| Fₛ = Iₛ·s·kₛ | |
| Fₛ = Schneidkraft | kₛ ≈ 0.8·Rₘ |
| Iₛ = Schnittlinienlänge | Rₘ = Zugfestigkeit |
| s = Schnittdicke/-durchmesser | |
| kₛ = Scherfestigkeit | |

Die oben angegebene Formel gilt dabei für einen 90°-Schnitt bei festem Untergrund.

Die Messungen werden dabei an einer Universalprüfmaschine mit Schneidkraftadapter mit vier Gleitlagern durchgeführt und mittels der Software TestXpert erfasst.

Für die Messung wurden von fünf Haarfasern zerschnitten.

Aufgrund des Einfluss des Querschnitts der Haarfaser ist eine Vorgruppierung der zu zerschneidenden Haarfasem unabdingbar, die sicherstellt, dass die zu vergleichenden Produkte an Haarfasem mit gleichem Querschnitt durchgeführt wurden, so dass relative Ergebnisse erzielt werden.

### Messaufbau:

Fünf einseitig fixierte Haarfasern werden mit Hilfe einer handelsüblichen Klinge (Einklingenrasierer mit Wechselklinge) bei einer konstanten Geschwindigkeit zerschnitten. Die Klinge ist dabei in einem eigens angefertigten Adapter eingespannt, der eine Führung der Klinge mit Hilfe von vier Gleitlagern sicherstellt. Die Bestimmung der Schneidkraft erfolgt über Maximalkraftdetektion. Gleichzeitig wird die Reibungskraft der Gleitlager während jeder Messung aufgenommen. Da die Schneidkräfte direkt abhängig von der Querschnittsfläche sind, ist vor jeder Messung sicherzustellen, dass die Verteilung der Haarfaserquerschnitte zwischen unterschiedlichen Gruppen vergleichbar ist.

| | | |
|---|---|---|
| Messparameter | Messgeschwindigkeit: | 100 mm/min |
| | Schneidewinkel: | 90° |
| | Klingenschliff: | ca. 12° |

Eine kosmetische Zubereitung, eine Rasurzubereitung, vermag die Gleit- und Schneidkräfte dabei zu beeinflussen.

Die getesteten Produkte A- D (s. Abbildung 1) umfassen eine Kunstschweißlösung bestehend aus:
Ausgangseluent: 1 Liter (Aqua dest)
   500 mg Glycin
   1300 mg Milchsäure (90 %aq)
   100 mg Harnstoff
   1800 mg NaCl
   480 mg KCI
   1000 mg Ammoniumchlorid
pH-Wert: ca. 3,0
   - basierend auf den Mittelwerten von Schweißanalysen männlicher und weiblicher Probanden -
sowie:
A: keine weiteren Bestandteile
B: Zitronensäure (wässrige Lösung, pH = 4,45)
C: Aluminiumchlorohydrat (ACH, 10 Gew. % in wässriger Lösung, pH = 4,45)
D: Aluminiumchlorohydrat (ACH, 10 Gew.% in wässriger Lösung, pH = 4,45) und Avocadoöl (0,1 Gew.%)

Die Zubereitungen C und D stellen bevorzugte erfindungsgemäße Zubereitungen dar.

Dass die Schneidkrafterniedrigung durch ACH nicht auf einem Einfluss des pH-Wertes beruht, belegt die Vergleichszubereitung B, in die Zitronensäure zugegeben wurde um zu einem ähnlichen pH-Wert wie die ACH-haltigen Zubereitungen zu gelangen.

Erstaunlich war die Erkenntnis, dass saure Aluminiumverbindungen zu einer Haarerweichung führen, was wiederum zu der gemessenen Schneidkrafterniedrigung führt.

Es ist bekannt, dass alkalische Zubereitungen, dass Haar aufweichen und damit eine vollständige Rasur ermöglichen. Bislang galten daher lediglich alkalische Zubereitungen, wie die bekannten Rasierseifen etc., als geeignet für eine gründliche Rasur.

Erfindungsgemäß sind nun aber auch überraschenderweise saure Aluminiumverbindungen in kosmetischen Zubereitungen als Rasurmittel geeignet.

Sauer bedeutet erfindungsgemäß ein pH-Wert in wässrigen Milieu von 7 und weniger.

Es sind somit saure Aluminiumverbindungen, insbesondere Aluminiumchlorohydrate, zur Herstellung von topisch applizierbaren Zubereitungen zur Erweichung des menschlichen Haares, insbesondere der Achselbehaarung, zu verwenden.

Ebenso ist die Verwendung von sauren Aluminiumverbindungen, insbesondere Aluminiumchlorohydrate, zur Herstellung von Zubereitungen zur Erniedrigung der Rasurschneidkraft nach topischer Applikation der Zubereitung, predestiniert zu verwenden.

Grundlage ist, dass die erfindungsgemäßen Zubereitungen umfassend saure Aluminiumsalze, insbesondere Aluminiumchlorohydrate, die Schneidkraft beim Rasieren verringern.

Werden der erfindungsgemäßen Zubereitung zusätzlich hautpflegende oder hauterweichende Zusätze zugegeben, wie beispielsweise Öle, zeigt sich eine nochmals verbesserte Rasur, bzw. Erniedrigung der Schneidkraft beim Rasieren (s. D, Abbildung 1).

Aluminiumchlorohydrate sind bekannte Antitranspirantmittel. Diese aber nun dazu zu verwenden, die sie enthaltende Zubereitungen auf die zu rasierenden Hautpartien zu applizieren um eine Rasur vollkommener zu gestalten, ist erstaunlich und nicht vorhersehbar gewesen.

Durch eine gründlichere Rasur ist die Häufigkeit, die Frequenz, mit der eine erneute Rasur stattfinden muss, somit erniedrigt.

Der Zusatz an Ölen, insbesondere Avocadoöl, in kosmetischen Zubereitungen ist bekannt. Erfindungsgemäß führt der Zusatz aber zu einer nochmals verbesserten Gründlichkeit, sowie Schneidkraftreduktion.

Avocadoöl gilt als geschmeidig machend und glättet sie. Als Ölkomponenten sind besonders vorteilhaft Avocadoöl und/oder die Lipide Octyldodecanol, Caprylyl Carbonate und/oder Paraffinum Liquidum in den erfindungsgemäßen Zubereitungen enthalten.

Vorteilhaft können zusätzlich Substanzen zur Verringerung des Haarwachstums der Zubereitung zugesetzt werden. Bevorzugte Substanzen sind aus der Gruppe Papaya-Extrakt, Carica Papaya, Schöllkraut-Extrakt und/oder Chelidonium Majus Extract zu wählen.

Die erfindungsgemäßen Zubereitungen sind bevorzugt emulsionsbasierende Zubereitungen. Auch als Lotion, Creme, Spray, Aerosol, als Roll-on oder Stick oder schaumförmige Zubereitungen lassen sich die erfindungsgemäßen Zubereitungen bereit stellen.

Bevorzugt werden die Zubereitungen als Aerosol, Roll-on oder Stick formuliert und bereit gestellt. Die dazu jeweils notwendigen Begleitsubstanzen, wie Treibgase bei Aerosolen, kann der Fachmann aus den bekannten Mitteln wählen.

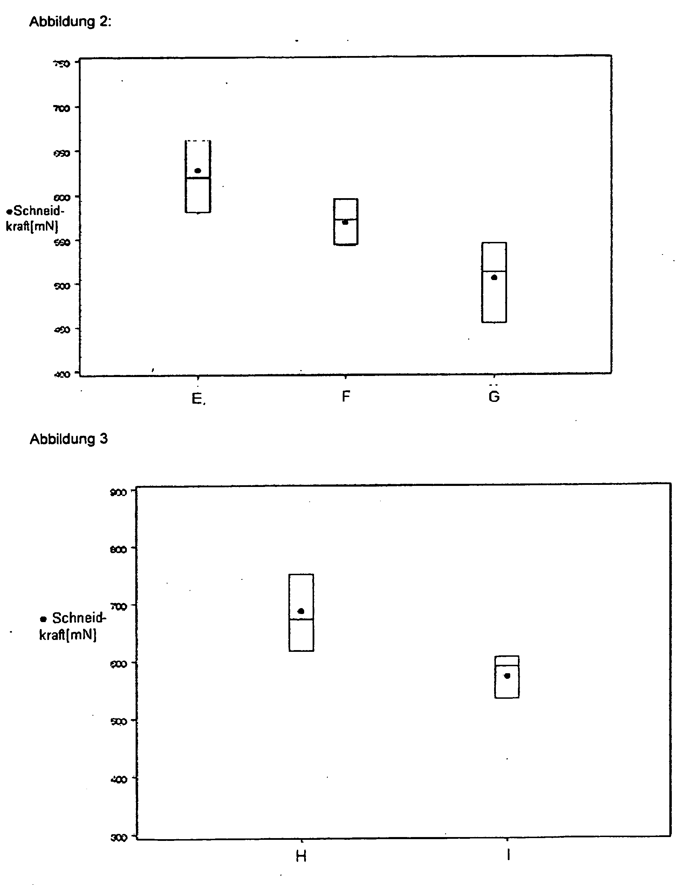

In einem weiteren Vergleichstest wurde die Schneidkraft an Humanhaar nach 24 stündiger Einwirkzeit von drei Zubereitungen gemessen (Abbildung 2).

Die getesteten Zubereitungen E - G (s. Abbildung 2) umfassen eine reine Kunstschweißlösung (wie Zubereitung A).
E: kein weiteren Bestandteile
F und G: zusätzlich eine emulsionsbasierende Zubereitung als Roll-on mit
F: als Makroemulsion

| | % (w/w) |
|---|---|
| Aqua | ad 100 |
| Aqua + Trisodium EDTA | 1.5 |
| Persea Gratissima Oil | 0,25 |
| Steareth-21 | 1,5 |
| Steareth-2 | 2,5 |
| PPG-15 Stearyl Ether | 3 |
| Aluminum Chlorohydrate (50 % aq) | 20 |
| Parfum | 1 |

G: als Mikroemulsion mit Paraffinöl

| | % (w/w) |
|---|---|
| Aqua | ad 100 |
| Octyldodecanol | 3 |
| Paraffinum Liquidum | 3 |
| Persea Gratissima Oil | 0,1 |
| Glyceryl Isostearate | 2 |
| Glycerin | 2,6 |
| Aluminum Chlorohydrate (50 % aq) | 20 |
| PEG-150 Distearate | 0,7 |
| Isoceteth-20 | 4 |
| Parfum | 1 |

Die Zubereitungen F und G stellen bevorzugte erfindungsgemäße Zubereitungen dar und zeigen die vorteilhafte Verwendung emulsionsbasierender Zubereitungen mit sauren Aluminiumverbindungen zur Haarerweichung, zur Schneidkraftreduktion und zur Verringerung der Frequenz der Rasur.

In einem weiteren Vergleichstest wurde die Schneidkraft an Humanhaar nach 24 stündiger Einwirkzeit von drei Zubereitungen gemessen (Abbildung 3).

Die getesteten Zubereitungen H - I (s. Abbildung 3) umfassen
H: eine reine eine Kunstschweißlösung (wie A)
I: zusätzlich eine emulsionsbasierende Zubereitung als Roll-on mit
I: als Mikroemulsion mit Dicraprylyl Carbonate

| INCl | % (w/w) |
|---|---|
| Aqua | ad 100 |
| Octyldodecanol | 3 |
| Persea Gratissima Oil | 0,1 |
| Glyceryl Isostearate | 2 |
| Glycerin | 2,6 |
| Aluminum Chlorohydrate | 20 |
| PEG-150 Distearate | 0,7 |
| Isoceteth-20 | 4 |
| Dicaprylyl Carbonate | 3 |
| Parfum , | 1 |

Die Zubereitung I stellt eine bevorzugte erfindungsgemäße Zubereitungen dar.

Bevorzugte Zubereitungen sind emulsionsbasierende Zubereitung, insbesondere als Makro- oder Mikroemulsion formuliert, vorteilhaft mit zusätzlichem Paraffinöl und/oder Dicraprylyl Carbonate.

Vorteilhaft sind die erfindungsgemäßen Zubereitungen als Roll-on zu verwenden.

Daneben ist auch die Verwendung als W/O-Spray erfindungsgemäß bevorzugt.

Die Zubereitungen sind aufgrund der Hautverträglichkeit bevorzugt alkoholfrei, tensidfrei und/oder seifenfrei.

Als tensidfrei bedeutet dabei nicht der Verzicht auf Emulgatoren.

Rasiermittel sind Trocken- oder Nassrasurapparate, metallische Klingen oder Messer.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse, sofern nicht ausgeschlossen, oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Moisturizer können vorteilhaft auch als Antifaltenwirkstoffe zum Schutz vor Hautveränderungen, wie sie z. B. bei der Hautalterung auftreten, verwendet werden.

Auf Bestandteile, die Penetrationsförderung für potenziell irritative oder allergene Bestandteile darstellen, wie beispielsweise ethoxylierten Polywachse, wie z.B. PEG-6000, kann erfindungsgemäß verzichtet werden.

Die erfindungsgemäßen Zubereitungen umfassend insbesondere antitranspirant wirksame Aluminiumverbindungen, wie Aluminiumchlorohydrate, führen neben der dargelegten Rasurfrequenzerniedrigung, Schneidkraftreduktion und damit zu einer hautschonenderen, Rasur auch zu einer Reduktion der Schweißbildung.

Es werden somit mit einem einzigen Präparat mehrere kosmetische Aufgaben erfüllt. Neben einer durchschnittlich 24 stündigen AT-wirksamkeit wird parallel die Haut für eine gründlicherer Rasur vorbereitet und nebenbei die Haut gepflegt, insbesondere wenn Öle, wie Avocadoöl, Octyldodecanol, Caprylyl Carbonate und/oder Paraffinum Liquidum enthalten sind.

Auftragungsort der erfindungsgemäßen Zubereitungen ist bevorzugt die Achselregion, die Beine sowie der Genital- und Brustbereich des Menschen.

Nachfolgende Beispiele zeigen bevorzugte Ausführungsformen der erfindungemäßen Formulierungen. Soweit nichts anderes angegeben, beziehen sich die Zahlenwerte auf Gewichtsanteile, bezogen auf die Gesamtmasse der Formulierungen.

| Beispiel | 1 |
|---|---|
| Aqua | 63,6 |
| Octyldodecanol | 3 |
| Persea Gratissima Oil | 0,1 |
| Glyceryl Isostearate | 2 |
| Glycerin | 2,6 |
| Aluminum Chlorohydrate | 20 |
| PEG-150 Distearate | 0,7 |
| Isoceteth-20 | 4 |
| Dicaprylyl Carbonate | 3 |
| Parfum | 1 |

### Beispiel 2: Roll-on (Emulsion, milchig weiß)

| | % (w/w) |
|---|---|
| Aqua | ad 100 |
| Aqua + Trisodium EDTA | 1,5 |
| Persea Gratissima Oil | 0,25 |
| Steareth-21 | 1,5 |
| Steareth-2 | 2,5 |
| PPG-15 Stearyl Ether | 3 |
| Aluminum Chlorohydrate (50 % aq) | 20 |
| Parfum | 1 |

### Beispiel 3: Roll-on (Emulsion, transzluzent)

| | % (w/w) |
|---|---|
| Aqua | ad 100 |
| Octyldodecanol | 3 |
| Paraffinum Liquidum | 3 |
| Persea Gratissima Oil | 0,1 |
| Glyceryl Isostearate | 2 |
| Glycerin | 2,6 |
| Aluminum Chlorohydrate (50 % aq) | 20 |
| PEG-150 Distearate | 0,7 |
| Isoceteth-20 | 4 |
| Parfum | 1 |

### Beispiel 4: Roll-on (Emulsion, transzluzent)

| | % (w/w) |
|---|---|
| Aqua | ad 100 |
| Octyldodecanol | 3 |
| Persea Gratissima Oil | 0,1 |
| Glyceryl Isostearate | 2 |
| Glycerin | 2,6 |
| Aluminum Chlorohydrate (50 % aq) | 20 |
| PEG-150 Distearate | 0,7 |
| Isoceteth-20 | 4 |
| Dicaprylyl Carbonate | 3 |
| Parfum | 1 |

### Beispiel 5: W/O-Spray (Aerosol)

| | % (w/w) in WL |
|---|---|
| Aqua | ad 100 |
| Preservative | qs |
| Persea Gratissima Oil | 0,5 |
| C12-15 Alkyl Benzoate | 5 |
| Cetyl PEG/PPG-10/1 Dimethicone | 1,5 |
| Aluminum Chlorohydrate (50 % aq) | 15-30 |
| Polysorbate 65 | 1 |
| Dicaprylyl Ether | 4 |
| Polyglyceryl-2 Dipolyhydroxystearate | 1,5 |
| Butyloctanoic Acid | 0,25 |
| Cyclomethicone | 11,75 |
| Dicaprylyl Carbonate | 5 |
| Parfum | qs |
| Abfüllung als Aerosol mit Treibgas (Propan/Butan). | |

### Beispiel 6: AT-Stick

| | % (w/w) |
|---|---|
| Octyldodecanol | 0,1 |
| Glyceryl Stearate SE | 0,6 |
| Persea Gratissima Oil | 0,5 |
| Hydrogenated Castor Oil | 1,5 |
| Talc | 4 |
| PPG-14 Butyl Ether | 15 |
| Cyclomethicone | ad 100 |
| Aluminum Zirconium Tetrachlorohydrex | |
| GLY | 10-20 |
| Stearyl Alcohol | 20 |
| Parfum | qs |

## Patentansprüche

1. Verwendung von sauren Aluminiumverbindungen als Wirkstoff zur Verringerung der Rasurfrequenz in topisch applizierbaren Zubereltungen.

2. Verwendung von sauren Aluminiumverbindungen als Wirkstoff zur Erweichung des menschlichen Haares, insbesondere der Achselbehaarung in topisch applizierbaren Zubereitungen.

3. Verwendung von sauren Aluminiumverbindungen als Wirkstoff zur Erniedrigung der Rasurschnetdkraft in topisch applizierbaren Zubereitungen.

4. Verwendung nach einem der vorstehenden Ansprüche, **dadurch gekennzeichnet, dass** der Zubereitung zusätzlich ein oder mehrere Lipide zugesetzt sind, bevorzugt gewählt aus der Gruppe Avocadoöl, Octyldodocanol, Caprylyl Carbonate und/oder Paraffinum Liquidum.

5. Verwendung nach Anspruch 4, indem Avocadoöl der Zubereitung zugesetzt wird.

6. Verwendung nach einem der vorangegangenen Ansprüche, Indem die Zubereitungen alkoholfrei, tensidfrei und/oder seitenfrei sind.

7. Verwendung nach einem der vorstehenden Ansprüche indem die Zubereitungen auf Basis einer Emulsion formuliert sind.

8. Verwendung nach einem der vorstehenden Ansprüche, **dadurch gekennzeichnet, dass** als saure Aluminiumverbindungen antitranspirant wirksame Aluminiumverbindungen, Insbesondere Aluminiumchlorohydrate, gewählt werden.

9. Verwendung nach einem der vorstehenden Ansprüche, **dadurch gekennzeichnet, dass** die Zubereitung als Aerosol, Roll-on oder Stick formuliert und bereit gestellt wird.

## Claims

1. Use of acidic aluminium compounds as active ingredient for reducing shaving frequency in topical preparations.

2. Use of acidic aluminium compounds as active ingredient for softening human hair, especially armpit hair, in topical preparations.

3. Use of acidic aluminium compounds as active ingredient for reducing the shaving cutting force in topical preparations.

4. Use according to one of the preceding claims, **characterized in that** one or more lipids are additionally added to the preparation, preferably selected from the group consisting of avocado oil, octyldodecanol, caprylyl carbonate and/or Paraffinum Liquidum.

5. Use according to Claim 4, wherein avocado oil is added to the preparation.

6. Use according to one of the preceding claims, wherein the preparations are alcohol-free, surfactant-free and/or soap-free.

7. Use according to one of the preceding claims, wherein the preparations are formulated on the basis of an emulsion.

8. Use according to one of the preceding claims, **characterized in that** antiperspirant aluminium compounds, in particular aluminium chlorohydrates, are selected as acidic aluminium compounds.

9. Use according to one of the preceding claims, **characterized in that** the preparation is formulated and provided as an aerosol, roll-on or stick.

## Revendications

1. Utilisation de composés acides d'aluminium en tant que substance active pour la diminution de la fréquence de rasage, dans des préparations applicables localement.

2. Utilisation de composés acides d'aluminium en tant que substance active pour l'atténuation de la pilosité humaine, en particulier de la pilosité des aisselles, dans des préparations applicables localement.

3. Utilisation de composés acides d'aluminium en tant que substance active pour la diminution de la force de coupe au rasage, dans des préparations applicables localement.

4. Utilisation selon l'une quelconque des revendications précédentes, **caractérisée en ce qu'**on ajoute en outre à la préparation un ou plusieurs lipides, de préférence choisis dans le groupe constitué par l'huile d'avocat, l'octyldodécanol, le Caprylyl Carbonate et/ou le Paraffinum Liquidum.

5. Utilisation selon la revendication 4, dans laquelle on ajoute de l'huile d'avocat à la préparation.

6. Utilisation selon l'une quelconque des revendications précédentes, dans laquelle les préparations sont sans alcool, sans tensioactifs et/ou sans savon.

7. Utilisation selon l'une quelconque des revendications précédentes, dans laquelle les préparations sont formulées à base d'une émulsion.

8. Utilisation selon l'une quelconque des revendications précédentes, **caractérisée en ce qu'**on choisit comme composés acides d'aluminium des composés d'aluminium à activité antisudorale, en particulier des chlorhydrates d'aluminium.

9. Utilisation selon l'une quelconque des revendications précédentes, **caractérisée en ce que** la préparation est formulée et produite sous forme d'aérosol, de roll-on ou de bâton.
